# FASCICULE DE BREVET EUROPEEN

(11) **EP 3 952 978 B1**
(45) Date de publication et mention de la délivrance du brevet: **19.02.2025**
(21) Numéro de dépôt: 20726301.3
(22) Date de dépôt: 07.04.2020
(51) Int. Cl.: A61M 39/28

(54) **DISPOSITIF DE PINCEMENT POUR APPAREIL MEDICAL**
KLEMMVORRICHTUNG FÜR MEDIZINISCHES INSTRUMENT
CLAMPING DEVICE FOR MEDICAL APPARATUS

(30) Priorité: 08.04.2019 FR 1903738
(43) Date de publication de la demande: 16.02.2022
(73) Titulaire: Maco Pharma, 59420 Mouvaux (FR)
(72) Inventeur: COASNE, Nicolas, 59320 Radinghem en Weppes (FR); BONTINCK, Pierre-Eloi, 59510 Forest-Sur-Marque (FR); CHAVATTE, Arnaud, 62350 Saint Floris (FR)
(74) Mandataire: Sayettat, Julien Christian
(86) Numéro de dépôt international: PCT/EP2020/059901
(87) Numéro de publication internationale: WO 2020/208022

(56) Documents cités:
- EP-A1- 0 624 382
- EP-A1- 3 103 489
- EP-B1- 0 624 382
- WO-A1-2017/063930
- US-A1- 2019 351 138

## Description

L'invention concerne un dispositif de pincement d'un tube souple ainsi qu'un système pour déterminer la présence ou l'absence d'un un procédé de détection d'un tube souple à l'aide d'un tel dispositif de pincement et un appareil comprenant un tel dispositif de pincement.

Elle s'applique au domaine médical, et notamment aux appareils utilisés pour le traitement du sang, tels que les appareils de collecte de sang.

Les prélèvements de sang sont habituellement réalisés à l'aide d'un système à poches comprenant au moins une poche de recueil du sang du donneur, reliée par l'intermédiaire d'un tube souple, à une aiguille de prélèvement. Lors de la collecte, l'aiguille de prélèvement est insérée dans la veine d'un donneur. La poche de recueil comprend un anticoagulant de type acide-citrate-dextrose (ACD) ou citrate-phosphate-dextrose (CPD) pour éviter la coagulation du sang.

Selon les standards européens, le volume de sang prélevé doit être compris entre 485 et 535 ml de sang, selon le sexe, la taille et le poids du donneur.

Il existe sur le marché des appareils automatisés pour la collecte du sang. Ces appareils comprennent typiquement un plateau agitateur sur lequel repose la poche de recueil du sang et un dispositif de pincement du tube souple reliant la poche de recueil et l'aguille de prélèvement. L'appareil est programmé pour pincer le tube souple lorsque le volume de sang à prélever est atteint afin d'arrêter le prélèvement.

Pour le bon fonctionnement de ces appareils, il est important que le tube souple à pincer soit présent et correctement positionné dans le dispositif de pincement.

Le document WO 2016/084001 décrit un exemple d'appareil automatisé de collecte de sang. Dans cet appareil, le dispositif de pincement pour fermer le tube souple est configuré pour définir trois positions : une position ouverte permettant l'insertion du tube souple et son retrait, une position de maintien, dans lequel le tube souple est maintenu dans le dispositif de pincement sans le fermer et une position fermée, dans laquelle le dispositif de pincement est fermé et compresse le tube souple pour stopper le flux de sang. D'autre part, ce dispositif de pincement peut comprendre en outre un détecteur de présence de tube souple.

Dans le document WO 2017/216343, il est décrit un appareil de collecte qui comprend un dispositif de pincement pourvu d'un capteur. Le capteur permet de détecter le degré d'ouverture de la fente dans laquelle est placé le tube et ainsi de détecter l'une des trois positions - ouverte, de maintien ou fermé - du dispositif de pincement.

D'autres appareils médicaux sont également munis de dispositifs de pincement de tube souple. Par exemple, le document EP 3 290 065 décrit un appareil pour extraire les composants sanguins comprenant plusieurs dispositifs de pincement dans lesquels des détecteurs de tubes, optiques ou mécaniques, sont agencés dans la fente recevant le tube à pincer.

Dans un autre exemple, le document US 2019/0351138 décrit une pompe médicale dans laquelle des capteurs de pression placés dans la gouttière recevant le tube souple permettent de s'assurer que le tube souple est monté dans la pompe.

Aussi, le document EP 3 103 489 décrit un dispositif de pincement de tube souple pour circulateur extracorporel comprenant trois types de détecteurs : (i) un détecteur de couvercle sous la forme d'un photo-interrupteur pour déterminer l'ouverture ou la fermeture d'un couvercle, (ii) un détecteur de présence de tube sous forme d'un capteur de pression mécanique agencé dans la fente recevant le tube souple et (iii) un détecteur de mouvement du piston sous forme d'un capteur optique au niveau du piston pour déterminer une position ouverte dans laquelle le tube souple n'est pas pincé et une position fermée dans laquelle le tube souple est pincé.

L'invention propose un dispositif de pincement permettant de différentier une position sans tube souple et une position avec tube souple afin de déterminer la présence ou absence d'un tube souple dans ledit dispositif.

A cet effet et selon un premier aspect, l'invention propose un dispositif de pincement d'un tube souple destiné à déterminer la présence dudit tube souple dans ledit dispositif de pincement, comprenant :
- un élément d'appui comprenant une surface d'appui contre laquelle ledit tube souple est destiné à être pincé,
- un élément de pincement comprenant une surface de pincement faisant face à la surface d'appui, ledit élément de pincement étant mobile par rapport audit élément d'appui entre au moins une position d'ouverture selon laquelle le tube souple placé entre la surface d'appui et la surface de pincement est non déformé, une position d'obturation selon laquelle le tube souple placé entre la surface d'appui et la surface de pincement est obturé et une position de fermeture selon laquelle la surface d'appui et la surface de pincement sont en contact, et
- une unité de pilotage configurée pour mettre en oeuvre les opérations suivantes :
   - actionner l'élément de pincement de la position d'ouverture en direction de la position de fermeture jusqu'à blocage de l'élément de pincement,
   - détecter la position de l'élément de pincement ainsi bloqué,
   - déterminer la présence ou l'absence du tube souple en fonction de la position de l'élément de pincement détectée.

Selon un deuxième aspect, l'invention concerne un système pour déterminer la présence ou l'absence d'un tube souple dans un dispositif de pincement comprenant un dispositif de pincement selon le premier aspect avec ledit tube souple.

Selon un troisième aspect, l'invention porte sur un procédé pour déterminer la présence ou l'absence d'un tube souple dans un dispositif de pincement selon le premier aspect, ledit procédé comprenant les opérations suivantes :
- déplacer l'élément de pincement de la position d'ouverture vers la position de fermeture,
- détecter la position de l'élément de pincement ainsi déplacé, et
- déterminer la présence ou l'absence du tube en fonction de la position de l'élément de pincement détectée.

Un quatrième aspect de l'invention concerne un appareil comprenant un dispositif de pincement selon le premier aspect de l'invention.

D'autres objets et avantages apparaîtront au cours de la description qui suit.

Les dessins annexés illustrent l'invention :
[Fig. 1] représente une vue schématique de face d'un dispositif de pincement selon une réalisation dans une position de grande d'ouverture.
[Fig. 2] représente une vue schématique partielle du dispositif de pincement de la Fig. 1 dans une position de grande ouverture.
[Fig. 3] représente une vue schématique partielle du dispositif de pincement de la Fig. 1 dans une position de petite ouverture.
[Fig. 4] représente une vue schématique partielle du dispositif de pincement de la Fig. 1 dans une position d'obturation.
[Fig. 5] représente une vue schématique partielle du dispositif de pincement de la Fig. 1 dans une position de fermeture.
[Fig. 6] représente une vue schématique partielle et en perspective du dispositif de pincement de la Fig. 1.
[Fig. 7] représente une vue schématique partielle et en coupe du dispositif de pincement de la Fig. 1.
[Fig. 8] représente une vue schématique de dessus de l'élément d'appui du dispositif de pincement de la Fig. 1
[Fig. 9] représente une vue schématique et en perspective d'un tube souple pincé.
[Fig. 10] représente une vue schématique d'un dispositif de pincement selon une autre réalisation.
[Fig. 11] représente une vue schématique d'un appareil médical comprenant un dispositif de pincement de la Fig. 1.
[Fig. 12] représente une vue schématique d'un système à poches pouvant être utilisé avec l'appareil de la figure 11.

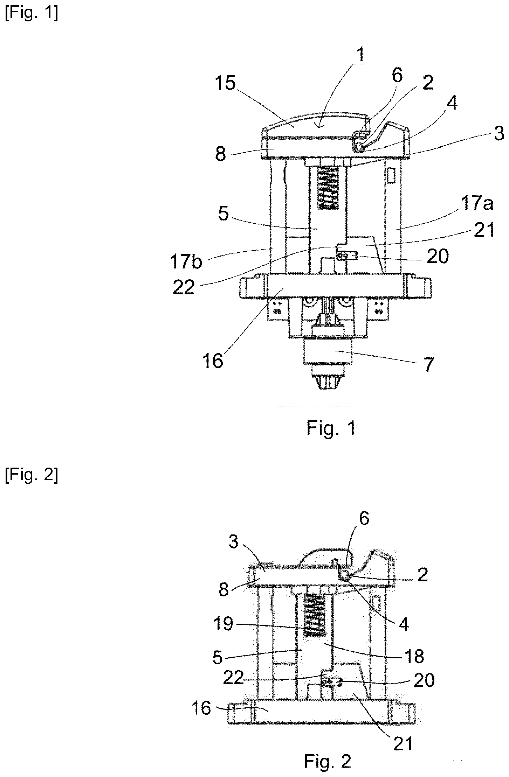

La figure 1 représente un dispositif de pincement selon une première réalisation. Ce dispositif de pincement est prévu pour faire partie d'un appareil médical, notamment d'un appareil pour la collecte du sang d'un donneur ou d'un patient. Ce dispositif de pincement 1 est destiné à pincer un tube souple 2 jusqu'à obturer une section de passage dudit tube souple et ainsi empêcher le passage d'un fluide au travers de ladite section.

Ce dispositif de pincement comprend :
- un élément d'appui 3 comprenant une surface d'appui 4 contre laquelle un tube souple 2 est destiné à être pincé, et
- un élément de pincement 5 comprenant une surface de pincement 6 faisant face à la surface d'appui 4, ledit élément de pincement 5 étant mobile par rapport audit élément d'appui 3 entre au moins une position d'ouverture, une position d'obturation et une position de fermeture.

L'expression « position d'ouverture » correspond à la position selon laquelle le tube souple placé entre la surface d'appui et la surface de pincement du dispositif de pincement est non déformé. Dans cette position, représentée sur les figures 1 à 3, la surface d'appui 4 et la surface de pincement 6 sont éloignées l'une de l'autre d'une distance supérieure ou égale au diamètre extérieur du tube souple à pincer.

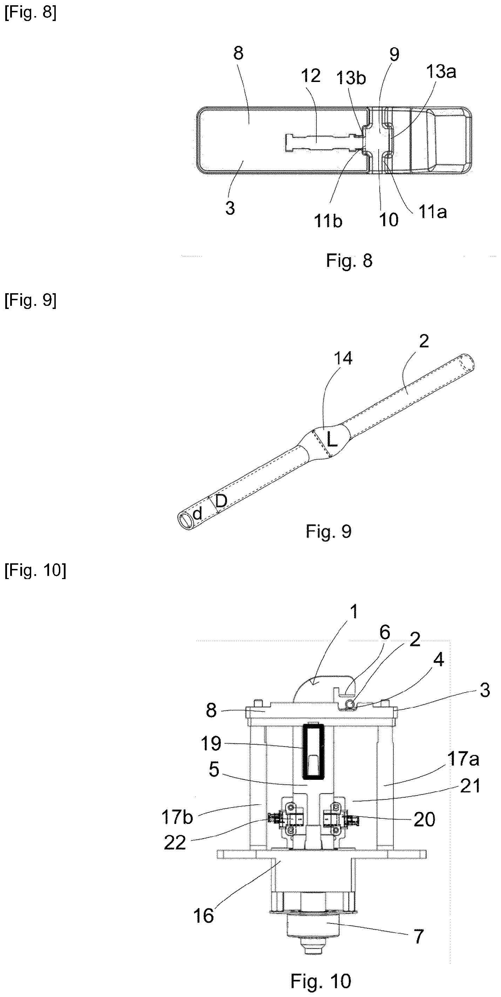

Lorsque la distance entre la surface d'appui 4 et la surface de pincement 6 est supérieure au diamètre extérieur D du tube souple 2 (figure 9), la position d'ouverture est dite « position de grande ouverture ». Cette position, représentée sur les figures 1 et 2, permet par exemple à un opérateur d'insérer le tube souple 2 dans le dispositif de pincement 1, entre la surface d'appui 4 et la surface de pincement 6.

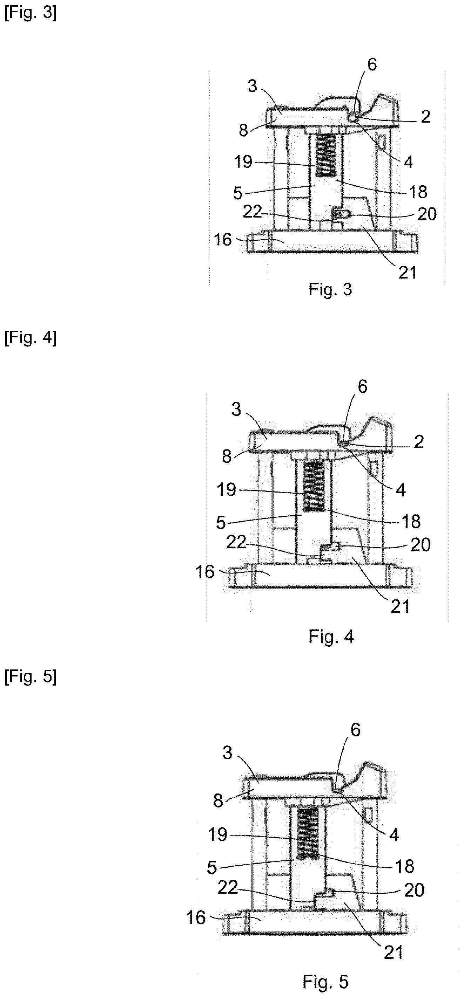

Lorsque la distance entre la surface d'appui 4 et la surface de pincement 6 est sensiblement égale au diamètre extérieur D du tube souple 2, la position d'ouverture sera dite « position de petite ouverture ». Dans cette position, représentée sur la figure 3, le tube souple est maintenu en place dans le dispositif de pincement par ajustement serré.

L'expression « position d'obturation » correspond à la position selon laquelle le tube souple 2 placé entre la surface d'appui 4 et la surface de pincement 6 du dispositif de pincement 1 est obturé. Dans cette position d'obturation, représentée sur la figure 4, une section de passage du tube souple 2 est fermée, empêchant le passage d'un fluide au travers de cette section. La distance entre la surface d'appui 4 et la surface de pincement 6 correspond substantiellement à deux fois l'épaisseur de la paroi du tube souple. En lien avec la figure 9, l'épaisseur de la paroi d'un tube souple 2 correspond à la différence entre le diamètre extérieur D du tube souple et le diamètre intérieur d dudit tube souple.

L'expression « position de fermeture » correspond à la position selon laquelle la surface d'appui 4 et la surface de pincement 6 du dispositif de pincement 1 sont en contact. Dans cette position de fermeture, représentée sur la figure 5, le tube souple est absent du dispositif de pincement.

Par exemple, pour un tube souple ayant un diamètre intérieur d de 3,5 mm et un diamètre extérieur D de 4,1 mm, la distance entre la surface d'appui 4 et la surface de pincement 6 du dispositif de pincement est sensiblement de 6 mm en position de grande ouverture, 4 mm en position de petite ouverture, 1,2 mm en position d'obturation, et 0 mm en position de fermeture.

Le dispositif de pincement 1 comprend en outre un organe d'entraînement 7 agencé pour produire un mouvement et le transmettre audit élément de pincement 5. Dans une réalisation particulière, l'organe d'entraînement 7 est configuré pour déplacer linéairement l'élément de pincement 5. En variante, l'élément de pincement est mobile en rotation.

Par exemple, l'organe d'entraînement est un moteur linéaire, particulièrement un moteur linéaire pas-à-pas. Alternativement, l'organe d'entraînement est un moteur à électro-aimants, un moteur à courant continu ou alternatif, un moteur sans balai, ou un vérin pneumatique ou hydraulique.

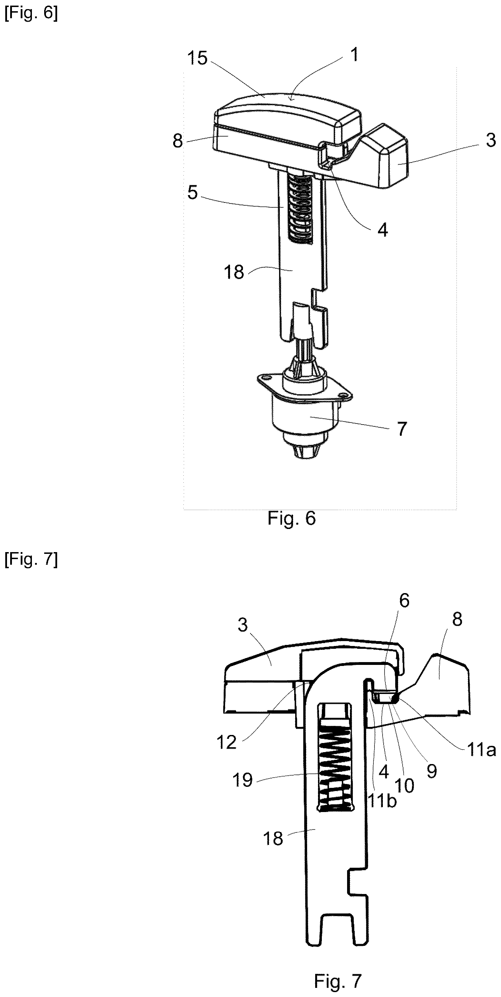

Selon une réalisation particulière représentée sur les figures 6 à 9, l'élément d'appui 3 comprend une première partie 8 avec un logement 9 pour ledit tube souple. Le logement 9 est sous forme d'un évidement comprenant un fond 10 substantiellement plat formant surface d'appui 4, et de deux parois latérales 11a,11b parallèles entre elles s'étendant à partir dudit fond 10. La largeur du logement correspond substantiellement au diamètre extérieur D du tube souple 2.

La première partie 8 de l'élément d'appui 3 comprend en outre une ouverture 12 pour le déplacement de l'élément de pincement décrit plus en détail ci-dessous. Cette ouverture 12 est notamment sous la forme d'une fente. L'ouverture 12 est isolée dudit logement 9 par l'une desdites parois latérales 11b du logement 9. Ainsi, en cas de fuite du fluide contenu dans le tube souple 2 au niveau du dispositif de pincement 1, le fluide s'écoule dans le logement 9 de l'élément d'appui 3 mais ne peut pas passer à l'intérieur du dispositif de pincement par l'ouverture 12. Le risque que le fluide s'écoule à l'intérieur du dispositif de pincement 1 au travers de l'ouverture 12 prévue pour l'élément de pincement 5 est donc réduit.

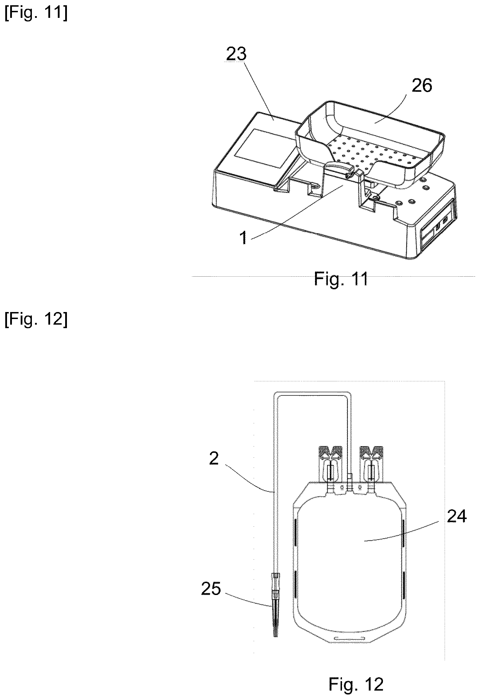

Avantageusement, le fond 10 du logement 9 présente une pente, par exemple entre 1 et 5 degré, par rapport à l'horizontal, afin de diriger le fluide pouvant s'écouler du tube souple vers l'extérieur de dispositif de pincement et/ou vers l'extérieur de l'appareil comprenant ledit dispositif de pincement. Par exemple, sur la figure 11, le bas de la pente est du côté opposé au plateau agitateur 26 de l'appareil de collecte 23.

Selon la figure 8, le logement 9 dudit tube souple comprend en outre une encoche 13a,13b sur chacune desdites parois latérales 11a,11b permettant l'écrasement du tube souple par l'élément de pincement.

En effet, comme illustré sur la figure 9, lorsqu'un tube souple est écrasé, la zone d'écrasement 14 possède une largeur L supérieure au diamètre extérieur D du tube souple.

Selon une réalisation particulière et en lien avec la figure 1, l'élément d'appui 3 comprend en outre un capot 15 destiné à être monté au-dessus de la première partie 8 du dispositif de pincement 1. Ce capot 15 protège l'élément de pincement 5 lorsqu'il est monté dans l'élément d'appui 3 afin d'éviter qu'un opérateur entrave le mouvement de l'élément de pincement lors du fonctionnement du dispositif de pincement.

L'élément d'appui 3 comprend une deuxième partie 16 solidaire de la première partie 8 par l'intermédiaire de deux tiges rigides 17a, 17b.

Sur la figure 1, l'organe d'entraînement 7 de l'élément de pincement 5 est fixé à la deuxième partie 16. L'élément de pincement 5 est solidaire de l'organe d'entrainement 7. L'élément de pincement 5 est arrangé entre la première et la deuxième partie 8,16 de l'élément d'appui 3.

L'élément de pincement 5 est mobile linéairement au travers de l'ouverture 12 de l'élément d'appui 3.

Selon les figures 6 et 7, l'élément de pincement 5 comprend un corps 18 sous forme d'une plaque couplée à une extrémité à l'organe d'entraînement 7 et comprenant à l'autre extrémité une portion recourbée se terminant par la surface de pincement 6.

Comme illustré sur la figure 7, l'élément de pincement 5 présente sensiblement la forme d'une crosse ou de la lettre J renversée. Cette forme particulière contribue à l'étanchéité du dispositif de pincement 1. En effet, lors du déplacement de l'élément de pincement 5, la surface de pincement 6 se déplace vers la surface d'appui 4 le long d'un axe distinct de l'axe dans lequel la plaque formant le corps 18 se déplace. L'ouverture 12 de l'élément d'appui 3, au travers de laquelle la plaque formant le corps de l'élément de pincement 5 se déplace, est isolée de la surface de pincement 6 et de la surface d'appui 4. En cas de fuite d'un liquide contenu dans le tube souple, le liquide ne peut pas passer au travers de l'ouverture 12 de l'élément d'appui et ne pénètre donc pas à l'intérieur du dispositif de pincement 1.

L'élément de pincement 5 comprend avantageusement un ressort 19 agencé en position initiale, pour placer l'élément de pincement 5 en position de fermeture. Sur la figure 7, le ressort est disposé dans une ouverture de la plaque formant le corps 18 de l'élément de pincement et vient en butée contre la première partie 8 de l'élément d'appui 3.

L'organe d'entraînement 7 est notamment un actionneur linéaire à moteur pas-à-pas pourvu d'un système anti-rotation tel que décrit dans le document US7969048.

Comme montré sur les figures 1 à 5, le dispositif de pincement 1 comprend avantageusement un détecteur de position 20 destiné à déterminer la position de l'élément de pincement 5, et en particulier la position d'obturation et la position de fermeture.

Ce détecteur de position 20 est éloigné de la surface d'appui 4 et de la surface de pincement 6 du dispositif de pincement 1, c'est-à-dire qu'il n'est pas au niveau du tube souple 2 à pincer. Le détecteur de position 20 détecte de façon indirecte la position de l'élément de pincement 5 par rapport à l'élément d'appui 3.

Notamment, le détecteur de position 20 est un capteur comprenant un émetteur d'un signal et un récepteur du signal émis. Ce capteur coopère avec l'élément de pincement 5 pour détecter la position dudit élément de pincement. Ce capteur est notamment un capteur sans contact de type optique, inductif, capacitif, magnétique ou ultrasonique.

Dans une configuration particulière, le détecteur de position 20 comprend un capteur optique. Le capteur est solidaire de l'élément d'appui 3. Comme montré sur la figure 1, le capteur formant détecteur de position 20 est notamment fixé sur un panneau 21 solidaire de la deuxième partie 16 de l'élément d'appui 3.

Selon les figures 1 à 5, l'élément de pincement 5 comprend une ouverture 22 qui coopère avec le capteur de manière à autoriser la réception par le récepteur du signal émis par l'émetteur lorsque l'élément de pincement est dans l'une des positions d'obturation ou de fermeture et de manière à empêcher la réception par le récepteur du signal émis par l'émetteur lorsque l'élément de pincement est dans l'autre position.

Par exemple, le signal est un signal optique de type faisceau lumineux. L'émetteur et le récepteur du capteur optique sont agencés d'un même côté de la plaque 21 de l'élément de pincement 5. Dans la position de fermeture (figure 5), l'émetteur et le récepteur du capteur optique sont en face de la partie pleine de la plaque et la lumière émise par l'émetteur est reçue par le récepteur. Dans la position d'obturation (figure 4), l'émetteur et le récepteur sont tous les deux en face de l'ouverture, et la lumière émise n'est reçue par le récepteur.

En particulier, l'ouverture est agencée pour empêcher la réception du signal par le récepteur dans la position de grande ouverture et de petite ouverture.

En variante, l'ouverture de l'élément de pincement est agencée pour autoriser partiellement la réception du signal par le récepteur dans la position d'obturation et pour empêcher la réception du signal dans les positions d'ouverture.

Selon une variante de réalisation représentée sur la figure 10, le dispositif de pincement 1 est conçu pour déterminer les quatre positions de l'élément de pincement 5, à savoir la position de grande ouverture, de petite ouverture, d'obturation et de fermeture.

Dans cette réalisation, le détecteur de position 20 comprend une paire de capteurs, qui coopèrent avec au moins une paire d'ouvertures 22 dans l'élément de pincement 5. Cette paire de capteurs et d'ouvertures permettent de déterminer la position de l'élément de pincement à l'aide d'une table de vérité.

Un exemple de table de vérité est la suivante, dans laquelle 1 correspond à la transmission de la lumière au récepteur du capteur optique et 0 correspond à l'absence de transmission de la lumière au capteur optique.

**[Tableau 1]**

| **Position** | **Capteur 1** | **Capteur 2** |
|---|---|---|
| Grande ouverture | 1 | 0 |
| Petite ouverture | 0 | 0 |
| Obturation | 1 | 1 |
| Fermeture | 0 | 1 |

Le dispositif de pincement 1 comprend en outre une unité de pilotage qui commande le déplacement de l'élément de pincement 5.

L'unité de pilotage se présente sous la forme d'un système électronique et informatique qui comprend par exemple un microprocesseur conçu pour exécuter un programme de commandes. L'exécution de ce programme permet à l'unité de pilotage de piloter notamment le dispositif de pincement, en fonction par exemple des signaux reçus par le détecteur de position.

Dans ce cas et avantageusement, afin de détecter la présence ou l'absence d'un tube souple 2 dans le dispositif de pincement 1, l'unité de pilotage est configurée pour :
- actionner l'élément de pincement 5 de la position d'ouverture en direction de la position de fermeture jusqu'à blocage de l'élément de pincement 5,
- détecter la position de l'élément de pincement ainsi bloqué, et
- déterminer la présence ou l'absence du tube souple en fonction de la position de l'élément de pincement détectée.

Le blocage de l'élément de pincement 5 se produit lorsque l'élément de pincement 5 est arrêté dans son déplacement, soit par la présence du tube souple 2 écrasé entre la surface d'appui 4 et la surface de pincement 6, soit par la surface d'appui 4 directement.

En particulier, la présence d'un tube souple 2 est déterminée si la position de l'élément de pincement détectée est la position d'obturation (figure 4). L'absence d'un tube est déterminée si la position de l'élément de pincement 5 détectée est la position de fermeture (figure 5).

L'unité de pilotage est configurée pour réagir différemment selon la présence ou l'absence de tube souple.

Par exemple, si le tube souple est absent du dispositif de pincement, l'unité de pilotage est configurée pour émettre un signal d'alerte sonore et/ou visuel à destination de l'opérateur pour l'alerter de l'absence de tube. De plus ou alternativement, l'unité de pilotage est configurée pour mettre en arrêt l'appareil comprenant le dispositif de pincement.

Si le tube souple est présent dans le dispositif de pincement, l'unité de pilotage est configurée pour poursuivre le fonctionnement de l'appareil.

Selon une réalisation avantageuse, l'unité de pilotage est en outre configurée pour vérifier, préalablement aux opérations de détection du tube souple, par exemple au démarrage de l'appareil comprenant le dispositif de pincement, que les différentes positions de l'élément de pincement sont correctement détectées. Cette opération de contrôle permet de s'assurer que lors des détections ultérieures, les cas de défaillances n'induisent pas de mauvaises réactions de l'unité de pilotage.

Par exemple, préalablement aux opérations de détection du tube souple et en l'absence de tube souple dans le dispositif de pincement, l'unité de pilotage est configurée, pour :
- actionner l'élément de pincement 5 de la position d'ouverture en direction de la position de fermeture jusqu'à blocage de l'élément de pincement
- détecter la position de l'élément de pincement 5 ainsi bloqué,
- déterminer le bon fonctionnement du dispositif de pincement 1 en fonction de la position de l'élément de pincement 5 détectée.

Si la position de l'élément de pincement détectée est la position de fermeture, le dispositif fonctionne correctement. Si la position de l'élément de pincement détectée n'est pas la position de fermeture, le dispositif de pincement est défectueux. Un signal d'alerte peut être émis à l'attention de l'opérateur.

Le dispositif de pincement 1 décrit ci-dessus avec un tube souple 2 forme un système pour déterminer la présence ou l'absence d'un tube souple dans un dispositif de pincement.

Il est maintenant décrit un procédé pour détecter un tube souple 2 dans un dispositif de pincement 1, ledit procédé comprenant les opérations consistant à :
- actionner l'élément de pincement 5 de la position d'ouverture en direction de la position de fermeture jusqu'à blocage de l'élément de pincement 5,
- détecter la position de l'élément de pincement 5 ainsi bloqué,
- déterminer la présence ou l'absence du tube souple 2 en fonction de la position de l'élément de pincement 5 détectée.

La présence d'un tube souple est déterminée si la position de l'élément de pincement détectée est la position d'obturation ; l'absence d'un tube souple est déterminée si la position de l'élément de pincement détectée est la position de fermeture.

Le dispositif de pincement tel que décrit ci-dessus est généralement compris dans un appareil.

Un tel appareil est notamment un appareil à usage médical qui peut être une pompe de perfusion, une soudeuse de tubes souples, un dispositif pour pincer et souder des tubes souples ou un appareil de traitement du sang. Dans un exemple particulier, l'appareil est un appareil pour la collecte du sang.

Un tel appareil 23 de collecte de sang est représenté sur la figure 11 et un exemple de système à poches pour le recueil du sang est représenté sur la figure 12.

Le système à poches comprend au moins une poche de recueil 24 pour collecter le sang, ladite poche de recueil étant reliée à une aiguille de prélèvement 25 par l'intermédiaire d'un tube souple 2. La poche de recueil 24 contient avantageusement une solution anticoagulante. L'aiguille de prélèvement est destinée à être introduit dans la veine d'un donneur afin de collecter son sang dans la poche de recueil.

Un système pour la collecte d'un volume cible de sang comprenant d'une part un appareil pour la collecte d'un volume cible du sang et d'autre part un système à poches comprenant une poche de recueil 24 et une aiguille de prélèvement 25 reliée à ladite poche de recueil par l'intermédiaire d'un tube souple, est décrit ci-après plus en détail.

L'appareil pour la collecte d'un volume cible de sang dans une poche de recueil d'un système à poches comprenant en outre une aiguille de prélèvement 25 reliée à ladite poche de recueil par l'intermédiaire d'un tube souple 2, comprend un plateau 26 destiné à recevoir la poche de recueil 24.

Avantageusement, l'appareil comprend en outre un dispositif d'agitation pour mettre en mouvement le plateau afin d'agiter la poche de recueil. L'agitation de la poche de recueil contribue à mélanger le sang collecté et la solution anticoagulante contenue dans la poche de recueil 24.

Un procédé particulier pour collecter un volume cible de sang dans une poche de recueil 24 d'un système à poches comprenant en outre une aiguille de prélèvement 25 reliée à ladite poche de recueil par l'intermédiaire d'un tube souple 2, comprend les étapes suivantes :
- déterminer le volume de sang collecté dans ladite poche de recueil 24, et
- pincer partielle le tube souple afin de réduire le débit du sang collecté, lorsque le volume déterminé de sang collecté dans ladite poche de recueil 24 atteint un volume prédéterminé.

Après l'étape de pincement partiel du tube souple, le procédé comprend notamment l'étape consistant à obturer le tube souple à l'atteinte du volume cible de sang à collecter afin d'arrêter la collecte de sang.

Pour notamment mettre en oeuvre ce procédé, l'appareil pour la collecte comprend, en plus du plateau 26 destiné à recevoir la poche de recueil, un dispositif de pincement 1 d'un tube souple tel que décrit ci-dessus et comprenant essentiellement :
(a) un élément d'appui 3 ayant une surface d'appui 4 contre laquelle ledit tube souple 2 est destiné à être pincé,
(b) un élément de pincement 5 ayant une surface de pincement 6 faisant face à la surface d'appui 4, ledit élément de pincement 5 étant mobile par rapport audit élément d'appui 3 entre au moins une position d'ouverture selon laquelle le tube souple placé entre la surface d'appui 4 et la surface de pincement 6 est non déformé et une position d'obturation selon laquelle le tube souple placé entre la surface d'appui 4 et la surface de pincement 6 est obturé, et
(c) une unité de pilotage configurée pour
   - déterminer le volume de sang collecté dans ladite poche de recueil 24, et
   - déplacer l'élément de pincement 5 de la position d'ouverture jusqu'à une première position intermédiaire entre ladite position d'ouverture et la position d'obturation, lorsque le volume déterminé de sang collecté dans ladite poche de recueil atteint un volume prédéterminé.

Le déplacement de l'élément de pincement jusqu'à une première position intermédiaire pince partiellement le tube souple en réduisant la section de passage dudit tube souple.

Ce pincement partiel du tube souple a pour effet de réduire le débit de sang collecté. Cette réduction du débit à la fin de la procédure de collecte de sang permet d'atteindre avec plus de précision le volume cible de sang à collecter, sans allonger de façon excessive le temps de collecte puisque la réduction du débit n'est mise en oeuvre qu'à l'approche de la fin de la procédure de collecte.

En particulier, le volume prédéterminé de sang collecté à partir duquel le débit est réduit, est compris dans la plage allant de 90 à 99 % du volume cible de sang à collecter, plus particulièrement dans la plage allant de 95 à 99 % du volume cible de sang à collecter. Cette prédétermination est réalisée par le dispositif de pilotage avant le début de la collecte du fluide.

Par exemple, pour un volume cible de 450 mL de fluide à collecter, le débit dans le tube souple est réduit lorsque le volume de fluide collecté atteint 440 mL.

La position intermédiaire est fixée par l'unité de pilotage. Elle peut être prédéterminée avant la collecte du sang, par exemple en fonction du volume cible de sang à collecter ou fixée pendant la collecte du sang, par exemple en fonction du débit réel de sang collecté.

Par exemple, la première position intermédiaire correspond substantiellement à la position médiane entre la position de petite ouverture et la position d'obturation.

En variante, l'unité de pilotage est configurée pour, après le déplacement de l'élément de pincement 5 à la première position intermédiaire, déplacer l'élément de pincement de la première position intermédiaire à une deuxième position intermédiaire entre ladite première position intermédiaire et la position d'obturation, lorsque le volume déterminé de sang collecté dans la poche de recueil atteint un autre volume prédéterminé de sang.de recueil.

Cette opération permet de réduire encore plus le débit de sang collecté à l'approche du volume cible de sang à collecter.

Le déplacement de l'élément de pincement dans ses différentes positions est mis en oeuvre par l'utilisation d'un organe d'entraînement 7 sous la forme d'un moteur pas-à-pas, qui selon le type de moteur, permet une précision de déplacement de l'ordre de 0,013 mm.

Afin de déterminer le volume de sang collecté dans la poche de recueil, l'appareil de collecte comprend en outre un dispositif pour mesurer le volume de sang collecté. En particulier, ce dispositif est un dispositif de pesée associé au plateau pour mesurer le poids de la poche de recueil. Le volume de sang collecté dans la poche de recueil est alors déduit du poids de la poche de recueil contentant le sang collecté.

A la mise en fonctionnement de l'appareil de collecte, l'unité de pilotage est configurée pour placer l'élément de pincement 1 dans une position de grande ouverture afin de permettre à l'opérateur de placer la tube souple 2 dans ledit dispositif de pincement 1. L'opérateur pose en outre la poche de recueil 24 et le reste du système à poches sur le plateau 26.

L'unité de pilotage est ensuite configurée pour déplacer l'élément de pincement 5 de la position de grande ouverture à la position d'obturation. Lorsque le tube souple est obturé, l'opérateur introduit l'aiguille dans une veine du donneur.

Ensuite, l'unité de pilotage est configurée pour déplacer l'élément de pincement de la position d'obturation à la position de petite ouverture afin de permettre la collecte du sang du donneur en maintenant le tube souple 2 dans le dispositif de pincement 1.

Avantageusement, juste après le déplacement de l'élément de pincement dans une position de grande ouverture, l'unité de pilotage est configurée pour mettre en oeuvre les opérations décrites ci-dessus permettant de déterminer la présence ou l'absence du tube souple 2 dans le dispositif de pincement 1. Le déplacement de l'élément de pincement 5 dans une position de petite ouverture pour permettre la collecte de sang n'est alors autorisé que si le tube souple est présent dans le dispositif de pincement.

Dans ce cas, le dispositif de pincement comprend en outre un détecteur de position 20 configuré pour détecter la position de l'élément de pincement 5, notamment la position d'obturation et la position de fermeture.

En cas d'absence de tube souple, l'unité de pilotage est configurée pour alerter l'opérateur de l'absence de tube. En cas de présence du tube souple, l'unité de pilotage est configurée pour démarrer la procédure de collecte du sang, notamment en déplaçant l'élément de pincement 5 dans la position de petite ouverture.

Lorsque le volume cible de sang est collecté dans la poche de recueil 24, l'unité de pilotage est configurée pour déplacer l'élément de pincement 5 de la position de petite ouverture à la position d'obturation à l'atteinte du volume cible de sang, afin d'arrêter la collecte du sang.

## Revendications

1. Dispositif de pincement (1) d'un tube souple (2) destiné à déterminer la présence dudit tube souple (2) dans ledit dispositif de pincement (1), comprenant :
- un élément d'appui (3) comprenant une surface d'appui (4) contre laquelle ledit tube souple (2) est destiné à être pincé,
- un élément de pincement (5) comprenant une surface de pincement (6) faisant face à la surface d'appui (4), ledit élément de pincement (5) étant mobile par rapport audit élément d'appui (3) entre au moins une position d'ouverture selon laquelle le tube souple placé entre la surface d'appui (4) et la surface de pincement (6) est non déformé, une position d'obturation selon laquelle le tube souple placé entre la surface d'appui (4) et la surface de pincement (6) est obturé et **caractérisé en ce que** ledit élément de pincement comprend aussi une position de fermeture selon laquelle la surface d'appui (4) et la surface de pincement (6) sont en contact, et caractérisé aussi **en ce que** le dispositf de pincement (1) comprend aussi:
- une unité de pilotage, ladite unité de pilotage étant configurée pour mettre en oeuvre les opérations suivantes :
- actionner l'élément de pincement (5) de la position d'ouverture en direction de la position de fermeture jusqu'à blocage de l'élément de pincement (5),
- détecter la position de l'élément de pincement (5) ainsi bloqué,
- déterminer la présence ou l'absence du tube souple (2) en fonction de la position de l'élément de pincement (5) ainsi détectée.

2. Dispositif selon la revendication 1, **caractérisé en ce que** la présence du tube souple (2) est déterminée si la position de l'élément de pincement (5) détectée est la position d'obturation, l'absence du tube souple (2) étant déterminée si la position de l'élément de pincement (5) détectée est la position de fermeture.

3. Dispositif selon l'une des revendications 1 ou 2, **caractérisé en ce que** l'unité de pilotage est en outre configurée pour émettre un signal d'alerte en cas d'absence de tube souple.

4. Dispositif selon l'une quelconque des revendications 1 à 3, **caractérisé en ce qu'**il comprend en outre un organe d'entraînement (7) configuré pour déplacer linéairement l'élément de pincement (5).

5. Dispositif selon la revendication 4, **caractérisé en ce que** l'élément de pincement (5) comprend un corps (18) sous forme d'une plaque couplée à une extrémité à l'organe d'entraînement (7) et comprenant à l'autre extrémité une portion recourbée se terminant par la surface de pincement (6).

6. Dispositif selon l'une quelconque des revendications 1 à 5, **caractérisé en ce qu'**il comprend en outre un ressort (19) agencé en position initiale pour placer l'élément de pincement (5) en position de fermeture.

7. Dispositif selon l'une quelconque des revendications 1 à 6, **caractérisé en ce qu'**il comprend en outre un détecteur de position (20) configuré pour détecter la position de l'élément de pincement (5), le détecteur de position (20) étant un capteur comprenant un émetteur de signal et un récepteur de signal qui coopère avec l'élément de pincement (5) pour détecter la position dudit élément de pincement.

8. Dispositif selon la revendication 7, **caractérisé en ce que** le détecteur de position (20) comprend un capteur optique.

9. Dispositif selon l'une des revendications 7 ou 8, **caractérisé en ce que** l'élément de pincement (5) comprend une ouverture (22) agencée de manière à autoriser la réception par le récepteur du signal émis par l'émetteur lorsque l'élément de pincement est dans l'une des positions d'obturation ou de fermeture, et de manière à empêcher la réception par le récepteur du signal émis par l'émetteur lorsque l'élément de pincement est dans l'autre position.

10. Dispositif selon l'une quelconque des revendications 7 à 9, **caractérisé en ce que** le détecteur de position (20) comprend une paire de capteurs.

11. Système pour déterminer la présence ou l'absence d'un tube souple (2) dans un dispositif de pincement (1) comprenant un dispositif de pincement (1) selon l'une quelconque des revendications 1 à 10 avec un tube souple (2).

12. Procédé pour déterminer la présence ou l'absence d'un tube souple (2) dans un dispositif de pincement selon l'une des revendications 1 à 10, ledit procédé comprenant les étapes suivantes :
- déplacer l'élément de pincement (5) de la position d'ouverture vers la position de fermeture,
- détecter la position de l'élément de pincement (5) ainsi déplacé,
- déterminer la présence ou l'absence du tube souple (2) en fonction de la position de l'élément de pincement (5) ainsi détectée.

13. Procédé selon la revendication 12, **caractérisé en ce que** la présence d'un tube souple (2) est déterminée si la position de l'élément de pincement (5) détectée est la position d'obturation, l'absence d'un tube souple (2) étant déterminée si la position de l'élément de pincement (5) détectée est la position de fermeture.

14. Appareil (23) comprenant un dispositif de pincement (1) selon l'une quelconque des revendications 1 à 10.

15. Appareil (23) selon la revendication 14 destiné à la collecte du sang, comprenant en outre un plateau agitateur (26) destiné à recevoir une poche de collecte (24).

## Patentansprüche

1. Vorrichtung zum Einklemmen (1) eines flexiblen Schlauchs (2), die dazu bestimmt ist, das Vorhandensein des flexiblen Schlauchs (2) in der Klemmvorrichtung (1) zu bestimmen, umfassend:
- ein Auflageelement (3), das eine Auflagefläche (4) umfasst, an der der flexible Schlauch (2) bestimmungsgemäß eingeklemmt werden soll,
- ein Klemmelement (5), das eine Klemmfläche (6) umfasst, die der Auflagefläche (4) zugewandt ist, wobei das Klemmelement (5) in Bezug auf das Auflageelement (3) zwischen mindestens einer Öffnungsposition, in der der zwischen der Auflagefläche (4) und der Klemmfläche (6) platzierte flexible Schlauch nicht verformt wird, einer Verschlussposition, in der der zwischen der Auflagefläche (4) und der Klemmfläche (6) platzierte flexible Schlauch verschlossen wird, bewegt werden kann, und **dadurch gekennzeichnet, dass** das Klemmelement ebenfalls eine Schließposition umfasst, in der sich die Auflagefläche (4) und die Klemmfläche (6) in Kontakt befinden, und ebenfalls **dadurch gekennzeichnet, dass** die Klemmvorrichtung (1) ebenfalls umfasst:
- eine Steuereinheit, wobei die Steuereinheit konfiguriert ist, um die folgenden Operationen umzusetzen:
- Betätigen des Klemmelements (5) aus der Öffnungsposition in Richtung der Schließposition, bis zum Sperren des Klemmelements (5),
- Erfassen der Position des so gesperrten Klemmelements (5),
- Bestimmen des Vorhandenseins oder Fehlens des flexiblen Schlauchs (2) in Abhängigkeit von der so erfassten Position des Klemmelements (5).

2. Vorrichtung nach Anspruch 1, **dadurch gekennzeichnet, dass** das Vorhandensein des flexiblen Schlauchs (2) bestimmt wird, wenn die erfasste Position des Klemmelements (5) die Verschlussposition ist, wobei das Fehlen des flexiblen Schlauchs (2) bestimmt wird, wenn die erfasste Position des Klemmelements (5) die Schließposition ist.

3. Vorrichtung nach einem der Ansprüche 1 oder 2, **dadurch gekennzeichnet, dass** die Steuereinheit weiter konfiguriert ist, um bei Fehlen des flexiblen Schlauchs ein Warnsignal zu senden.

4. Vorrichtung nach einem der Ansprüche 1 bis 3, **dadurch gekennzeichnet, dass** sie weiter ein Antriebsorgan (7) umfasst, das konfiguriert ist, um das Klemmelement (5) linear zu verlagern.

5. Vorrichtung nach Anspruch 4, **dadurch gekennzeichnet, dass** das Klemmelement (5) einen Körper (18) in Form einer Platte umfasst, die an einem Ende mit dem Antriebsorgan (7) gekoppelt ist und am anderen Ende einen gebogenen Abschnitt umfasst, der in der Klemmfläche (6) endet.

6. Vorrichtung nach einem der Ansprüche 1 bis 5, **dadurch gekennzeichnet, dass** sie weiter eine Feder (19) umfasst, die in der Ausgangsposition eingerichtet ist, um das Klemmelement (5) in der Schließposition zu platzieren.

7. Vorrichtung nach einem der Ansprüche 1 bis 6, **dadurch gekennzeichnet, dass** sie weiter einen Positionsdetektor (20) umfasst, der konfiguriert ist, um die Position des Klemmelements (5) zu erfassen, wobei der Positionsdetektor (20) ein Sensor ist, der einen Signalsender und einen Signalempfänger umfasst, der mit dem Klemmelement (5) zusammenwirkt, um die Position des Klemmelements zu erfassen.

8. Vorrichtung nach Anspruch 7, **dadurch gekennzeichnet, dass** der Positionsdetektor (20) einen optischen Sensor umfasst.

9. Vorrichtung nach einem der Ansprüche 7 oder 8, **dadurch gekennzeichnet, dass** das Klemmelement (5) eine Öffnung (22) umfasst, die so eingerichtet ist, dass sie den Empfang des vom Sender gesendeten Signals durch den Empfänger gestattet, wenn sich das Klemmelement in einer der Verschluss- oder der Schließposition befindet, und so, dass sie den Empfang des vom Sender gesendeten Signals durch den Empfänger verhindert, wenn sich das Klemmelement in der anderen Position befindet.

10. Vorrichtung nach einem der Ansprüche 7 bis 9, **dadurch gekennzeichnet, dass** der Positionsdetektor (20) ein Sensorpaar umfasst.

11. System, um das Vorhandensein oder Fehlen eines flexiblen Schlauchs (2) in einer Klemmvorrichtung (1) zu bestimmen, das eine Klemmvorrichtung (1) nach einem der Ansprüche 1 bis 10 mit einem flexiblen Schlauch (2) umfasst.

12. Verfahren, um das Vorhandensein oder Fehlen eines flexiblen Schlauchs (2) in einer Klemmvorrichtung nach einem der Ansprüche 1 bis 10 zu bestimmen, wobei das Verfahren die folgenden Schritte umfasst:
- Verlagern des Klemmelements (5) aus der Öffnungsposition zur Schließposition,
- Erfassen der Position des so verlagerten Klemmelements (5),
- Bestimmen des Vorhandenseins oder Fehlens des flexiblen Schlauchs (2) in Abhängigkeit von der so erfassten Position des Klemmelements (5).

13. Verfahren nach Anspruch 12, **dadurch gekennzeichnet, dass** das Vorhandensein eines flexiblen Schlauchs (2) bestimmt wird, wenn die erfasste Position des Klemmelements (5) die Verschlussposition ist, wobei das Fehlen eines flexiblen Schlauchs (2) bestimmt wird, wenn die erfasste Position des Klemmelements (5) die Schließposition ist.

14. Einrichtung (23), die eine Klemmvorrichtung (1) nach einem der Ansprüche 1 bis 10 umfasst.

15. Einrichtung (23) nach Anspruch 14, die zum Sammeln von Blut bestimmt ist, die weiter ein Schütteltablett (26) umfasst, das dazu bestimmt ist, einen Sammelbeutel (24) aufzunehmen.

## Claims

1. Device for clamping (1) a flexible tube (2) intended for determining the presence of said flexible tube (2) in said clamping device (1), comprising:
- a bearing element (3) comprising a bearing surface (4) against which said flexible tube (2) is intended for being clamped,
- a clamping element (5) comprising a clamping surface (6) facing the bearing surface (4), said clamping element (5) being able to move with respect to said bearing element (3) between at least one open position according to which the flexible tube placed between the bearing surface (4) and the clamping surface (6) is not deformed, an obturation position according to which the flexible tube placed between the bearing surface (4) and the clamping surface (6) is obstructed and **characterised in that** said clamping element also comprises a closed position according to which the bearing surface (4) and the clamping surface (6) are in contact, and also **characterised in that** the device for clamping (1) also comprises:
- a control unit, said control unit being configured to implement the following operations:
- actuating the clamping element (5) from the open position towards the closed position until the clamping element (5) is blocked,
- detecting the position of the clamping element (5) thus blocked,
- determining the presence or the absence of the flexible tube (2) according to the position of the clamping element (5) that has thus been detected.

2. Device according to claim 1, **characterised in that** the presence of the flexible tube (2) is determined if the position of the clamping element (5) detected is the obturation position, the absence of the flexible tube (2) being determined if the position of the clamping element (5) detected is the closed position.

3. Device according to one of claims 1 or 2, **characterised in that** the control unit is furthermore configured to emit an alert signal in case of absence of a flexible tube.

4. Device according to any of claims 1 to 3, **characterised in that** it further comprises a drive member (7) configured to linearly displace the clamping element (5).

5. Device according to claim 4, **characterised in that** the clamping element (5) comprises a body (18) in the form of a plate coupled at one end to the drive member (7) and comprising at the other end a curved portion ending with the clamping surface (6).

6. Device according to any of claims 1 to 5, **characterised in that** it further comprises a spring (19) arranged in the initial position to place the clamping element (5) in the closed position.

7. Device according to any of claims 1 to 6, **characterised in that** it further comprises a position detector (20) configured for detecting the position of the clamping element (5), the position detector (20) being a sensor comprising a signal transmitter and a signal receiver that cooperates with the clamping element (5) to detect the position of said clamping element.

8. Device according to claim 7, **characterised in that** the position detector (20) comprises an optical sensor.

9. Device according to one of claims 7 or 8, **characterised in that** the clamping element (5) comprises an opening (22) arranged so as to authorise the receiving by the receiver of the signal transmitted by the transmitter when the clamping element is in one of the obturation or closed positions, and in such a way as to prevent the receiver from receiving the signal transmitted by the transmitter when the clamping element is in the other position.

10. Device according to any of claims 7 to 9, **characterised in that** the position detector (20) comprises a pair of sensors.

11. System for determining the presence or the absence of a flexible tube (2) in a clamping device (1) comprising a clamping device (1) according to any of claims 1 to 10 with a flexible tube (2).

12. Method for determining the presence or the absence of a flexible tube (2) in a clamping device according to one of claims 1 to 10, said method comprising the following steps:
- displacing the clamping element (5) from the open position to the closed position,
- detecting the position of the clamping element (5) that has thus been displaced,
- determining the presence or the absence of the flexible tube (2) according to the position of the clamping element (5) that has thus been detected.

13. Method according to claim 12, **characterised in that** the presence of a flexible tube (2) is determined if the position of the clamping element (5) detected is the obturation position, the absence of a flexible tube (2) being determined if the position of the clamping element (5) detected is the closed position.

14. Apparatus (23) comprising a clamping device (1) according to any of claims 1 to 10.

15. Apparatus (23) according to claim 14 intended for collecting blood, further comprising an agitator plate (26) intended for receiving a collecting bag (24)
